# EUROPEAN PATENT APPLICATION

(11) **EP 4 717 142 A1**
(43) Date of publication of application: **01.04.2026**
(21) Application number: 24203637.4
(22) Date of filing: 30.09.2024
(51) Int. Cl.: A61B 1/00, A61B 1/005

(54) **BENDABLE TUBE FOR USE IN AN ENDOSCOPE**

(71) Applicant: Hoya Corporation, Tokyo 160-8347 (JP)
(72) Inventor: RIZZO, Marco, Tokyo, 160-8347 (JP); WALBERG, Erik, Tokyo, 160-8347 (JP)
(74) Representative: Lucke, Andreas

(57) **Abstract**

A tube for an endoscope, the tube comprising a continuous cylindrical body extending along an axial direction and defining an inner channel radially delimited by the body, the body comprising a plurality of cutout sections distributed along the axial direction in an active bending section of the tube, wherein the body is partially discontinuous at the cutout sections, such that uncut axial bridges connect proximal and distal sections of the body adjacent to the cutout sections, and wherein circumferential bridge sections of the body are formed between the cutout sections along the axial direction; wherein a subset of the circumferential bridge sections is bent radially inward to form bent circumferential bridge sections between neighboring cutout sections, wherein a lateral extension of the body perpendicular to the axial direction is reduced at the bent circumferential bridge sections.

## Description

### FIELD OF THE DISCLOSURE

The present disclosure is in the field of medical technology. In particular, the present disclosure relates to a bendable tube for use in an endoscope.

### BACKGROUND

An endoscope is a surgical device that may be used to access (e.g., view or remove) or treat tissue within the body of a patient by inserting one or more medical tools into the body through an incision in the body or an orifice of the body. The endoscope may include (comprise) an interface/control portion and an insertion tube that is coupled to the interface/control portion. The insertion tube is configured to be inserted into the body of the patient and may include one or more channels to provide access to tissue within the body. The one or more channels may e.g. be configured to receive a medical tool and/or a fluid and to guide the medical tool and fluid, respectively, to the tissue of interest.

The insertion tube may be bendable to facilitate insertion into the patient's body. For this, the insertion tube may for example include one or more passively and/or actively bendable sections (also referred to as passive and active bending section, respectively). A passively bendable section may e.g. be formed of a bendable or flexible material or structure that can be deformed by applying an external force.

An actively bendable section of the insertion tube may be provided for actively navigating a tip of the endoscope at a distal end of the insertion tube. Contrary to the passive bending section, the active bending section includes couplings for coupling to a control mechanism, generally provided in the form of wire(s) which transmit an external force applied along the wire(s) onto the active bending section, such as to deflect a distal section of the insertion tube in response to the applied force. The actively bendable section usually includes a plurality of puzzled joints that can be articulated to bend the actively bendable section, and flaps may be provided inside of the actively bendable section, which can feature through-holes guiding the wire(s) along the axial direction of the actively bendable section.

### SUMMARY OF THE DISCLOSURE

However, such actively bendable sections may be mechanically unstable as the joints may disconnect from each other, e.g. if there is slack in the control wires, and may require a complex assembly and low manufacturing tolerance. Moreover, the joint geometry may limit the available space inside of the insertion tube for medical tools and fluid channels extending from a tip of the insertion tube towards a proximal section of the insertion tube outside of a patient's body.

It is thus a feature of the present disclosure to provide a tube for use in an endoscope that exhibits improved mechanical stability and efficient torque transfer while also providing sufficient space inside the tube.

The present disclosure provides a tube for use in an endoscope and a manufacturing method thereof as set out in the appended independent claims. Examples thereof are detailed in the dependent claims.

According to a first aspect a tube for an endoscope is provided, the tube comprising a continuous cylindrical body extending along an axial direction and defining an inner channel radially delimited by the body. The body comprises a plurality of cutout sections distributed along the axial direction in an active bending section of the tube, wherein the body is partially discontinuous at the cutout sections, such that uncut axial bridges connect proximal and distal sections of the body adjacent to the cutout sections. Circumferential bridge sections of the body are formed between the cutout sections along the axial direction. A subset of the circumferential bridge sections is bent radially inward to form bent circumferential bridge sections between neighboring cutout sections, and a lateral extension of the body perpendicular to the axial direction is reduced at the bent circumferential bridge sections.

The tube may be or be part of an insertion tube, which can be actively angulated through an angulation mechanism, e.g. based on a pulling force transmitted through a control wire, and may define a hollow section for guiding medical tools and/or fluids along the axial extension of the insertion tube, e.g. from a proximal section towards a distal portion of the insertion tube. As used herein, the term "proximal" may refer to an element, a feature or a location along the length of the tube that is to be closer to a physician or other medical practitioner during use of the endoscope (e.g. closer to an interface/control portion of the endoscope), whereas the term "distal" may refer to an element, a feature or a location along the length of the tube that is to be closer to a location of tissue being examined or treated within the body of a patient during use of the endoscope (e.g. further away from an interface/control portion of the endoscope). A direction along the center line of the tube (e.g. from the proximal end to the distal end) may also be referred to as a longitudinal or axial direction in the following. A direction along a circumference of the tube (for example parallel to a surface, e.g. an outer surface, of the tube and perpendicular to the longitudinal direction) may also be referred to as a circumferential or azimuthal direction. A direction perpendicular to a surface, e.g. an outer surface, of the tube (for example perpendicular to the longitudinal direction and to the circumferential direction) may also be referred to as a radial direction. The longitudinal, circumferential and radial directions may for example define a cylindrical coordinate system. Depending on the state or configuration of the tube, the orientation of these directions may vary along the length of the tube, e.g. as a result of a bending of the tube.

The center line of the tube may extend along a center of the body. The center line may for example be a line or path connecting the centers (e.g. the centroids) of the outer or inner circumferences of the tube along the length of the body. Depending on the state or configuration of the tube, the center line of the tube may not be a straight line, but may e.g. follow a curved path.

The tube may feature a main bending mode, which may be aligned with the uncut axial bridges. The uncut axial bridges can be integral portions of the body and may act as hinges about which adjacent sections of the body can pivot to change a bending state of the body, with the uncut axial bridges being elastically deformed. The uncut axial bridges may be aligned along the circumferential direction, such as to define a common bending direction of the body, e.g. perpendicular to an extension direction of the uncut axial bridges between adjacent circumferential bridge sections. Thus, in response to an external force, the body can be bent at the uncut axial bridges, such that opposite axial edges of neighboring cutout sections approach each other to angulate the tube. Since the active bending section may be defined by a plurality of cutout sections, the active bending section may be integrally formed from the body.

In some examples, the tube further comprises a control wire extending axially through the active bending section towards a distal end of the active bending section, wherein pulling on a proximal portion of the control wire bends the active bending section of the tube with sections of the body pivoting about the uncut axial bridges.

The external force for angulating the body may be mediated by a control wire coupled to a distal end of the active bending section, wherein the control wire may run along an inner side wall of the body, such that increasing a tension along the control wire may induce a bending force on the body for bending towards the control wire. In some examples, two control wires are arranged on opposite sides of the body and tightening one of the control wires may bend the body towards the tightened control wire in the active bending section. Optionally, the other control wire may be relaxed to support the bending. The control wire(s) may be arranged at a different side of the body with respect to the axial bridge section, such that adjusting the tension of the control wire may promote bending of the body with the axial bridge sections acting as hinges.

The control wire may for example be configured to, when actuated, apply a compressive tension to the tube or a part thereof, in particular the active bending section, along the longitudinal direction, e.g. from the distal end to the proximal end. The control wire may for example be connected or attached to a distal attachment point, which may for example be arranged on a distal side of the tube, e.g. at or adjacent to the distal end or tip of the tube. When pulling on a proximal end of the control wire, a force may be generated from the distal attachment point towards the proximal end of the tube. In some examples, the control wire may also be configured to apply a compressive tension even when not actuated, for example to bias the tube towards the default configuration.

At the circumferential bridge sections, the body may extend continuously around a perimeter of the cylindrical body. Accordingly, the body may be considered to define a series of annular sections comprising the circumferential bridge portions and interconnected by the uncut axial bridges, wherein the annular sections may be considered to tilt in response to the external force about the uncut axial bridges.

The bent circumferential bridge sections are bent inwards towards a center line of the body and may provide supports for guiding the control wire(s) and/or for holding a sleeve of the control wire in place, e.g. at a proximal end of the active bending section.

The bent circumferential bridge sections may be defined by cutout sections on axially neighboring sections of the body, and may be inelastically deformed towards the center line. The bent circumferential bridge sections may feature kinks on opposite circumferential sides and may feature a concave outer surface close to a center of the bent circumferential bridge section. An outline of the bent circumferential bridge sections, e.g. when viewed from the side of the tube, may be the same as an outline of a neighboring (unbent) circumferential bridge section. The bent circumferential bridge section may be formed by application of an external pressing force for bending the respective circumferential bridge section towards the center line of the body, and the bent circumferential bridge section may generally be integrally formed with adjacent axial bridge sections and/or neighboring circumferential bridge sections.

The wire may run over an outer circumference of the bent circumferential bridge section, e.g. on an outer surface of the bent circumferential bridge section that faces radially outwards as seen from the center line of the tube.

In some examples, the circumferential bridge sections define wire guidance slots between an outer surface of the bent circumferential bridge sections and inner surfaces of neighboring circumferential bridge sections, to guide an control wire for actively angulating the tube.

The bent circumferential bridge section may in particular be arranged such that the control wire, when arranged in the guiding slot defined by the bent circumferential bridge, is located on the outside of tube, i.e. not in or facing the central cavity, which may form the inner channel of the tube.

The neighboring circumferential bridge sections may be not bent inwards towards the center line of the body, such that a radial gap formed between the outer surface of the bent circumferential bridge section and the inner surface of the unbent neighboring circumferential bridge portion may define a channel, through which the control wire may extend.

When viewed along the axial direction, a slot defined between the bent circumferential bridge section and an adjacent circumferential bridge section, which is not bent inwards, may taper from central portion towards uncut axial bridges arranged at circumferential ends of the bent circumferential bridge section, such as to center the control wire towards a center of the adjacent cutout sections in the circumferential direction.

In some examples, two bent circumferential bridge sections are arranged on opposite sides of the cylindrical body in a radial direction, to define wire guidance slots symmetrically disposed on opposite sides of the cylindrical body.

In some examples, pairs of cutout sections are distributed along the axial direction, the cutout sections of a pair of cutout sections being arranged on opposite sides of the cylindrical body in a radial direction, and wherein the cutout sections of the pair of cutout sections are separated by uncut axial bridges on opposite sides in the circumferential direction.

Accordingly, two circumferential bridge portions may be formed between neighboring pairs of cutout sections, on opposite sides of the body (in a radial direction), and the two circumferential bridge portions may be connected along the circumferential direction and may together define an annular section of the body extending continuously around a perimeter of the cylindrical body.

In some examples, the cutout sections comprise an angulation control portion, wherein the angulation control portion is arranged between circumferential ends of the cutout sections and features a width along the axial direction, which is smaller than a width of neighboring portions closer to the circumferential ends of the cutout sections.

The reduced width of the angulation control portion may increase a width of an adjacent circumferential bridge section in the axial direction, such as to limit local angulation of the cylindrical body through mechanical interference between adjacent circumferential bridge sections and/or to improve control wire guidance at an adjacent bent circumferential bridge section. For example, a width of a circumferential bridge section along the axial direction at a center of the circumferential bridge section may be greater than a width of the circumferential bridge section halfway between the center and the circumferential end of an adjacent cutout section, by at least 5 or at least 10%. As another example, a width of a cutout section along the axial direction at a center of the cutout section may be smaller than a width of the cutout section halfway between the center of the cutout section and the circumferential end of an cutout section, by at least 5 or at least 10%.

In some examples, the cutout sections at an axial position along the body cover more than 70% or more than 80% of the perimeter of the body.

For example, two cutout sections at a common axial position along the axial extension of the body may together cover more than 70% or more than 80% of the perimeter, such as to enable sufficient flexibility of the body for bending about an uncut axial bridge arranged between the two cutout sections.

The width of the uncut axial bridges along the circumferential direction may define an effective bending stiffness of the body in the active bending section, e.g. in response to an external force, which may be applied through the control wires.

In some examples, a width of the cutout sections along the axial direction is greater than a wall thickness of the body in the radial direction, and generally greater than twice or three times the wall thickness of the body.

In some examples, the width of the cutout sections along the axial direction along the axial direction is smaller than the width of the circumferential bridge along the axial direction, such as between 10% and 90% of the width of the circumferential bridge or between 20% and 80% of the width of the circumferential bridge along the axial direction. The width may be the average width of the cutout section/circumferential bridge section or the respective width at a circumferential center of the cutout section.

The cutout sections may further define a stop section along the axial direction of the body for holding a wire sleeve of the control wire in place.

In some examples, the tube further comprises a stop section arranged at a proximal portion of the active bending section and configured to prevent a wire sleeve of a control wire running through the stop section from moving towards the distal end of the active bending section, wherein the wire sleeve abuts against a bent circumferential bridge section in the stop section.

The stop section may be arranged at a proximal end of the active bending section, and may be integrally formed with the active bending section from the body. The stop section may be arranged between a passive bending section and the active bending section, with the passive bending section arranged proximally with respect to the stop section. In some examples, the passive bending section and the stop section are integrally formed from a common tube element. The control wire may run from a proximal portion of an endoscope, through the passive bending section, and through the stop section into the active bending section.

The control wire may be held in the wire sleeve to transfer a pulling force on the control wire from a proximal portion of the endoscope through the wire sleeve towards a distal end of the wire sleeve, such as to enable a surgeon to actively bend the active bending section from the proximal portion of the endoscope, e.g. without actively angulating the passive bending section. The control wire held in the wire sleeve may form a Bowden cable, and the distal end of the wire sleeve may abut against the stop section as a counter bearing for bending the active bending section of the body. The wire sleeve may comprise a hollow metal tube, e.g. formed from wound steel wire, for accommodating the control wire. The distal end of the wire sleeve can abut against a bent circumferential bridge section in the stop section to prevent the wire sleeve from moving further towards a distal end of the endoscope.

In some examples, the stop section comprises a bent circumferential bridge section arranged between a proximal axial slot and a distal axial slot defined by a proximal cutout section and a distal cutout section, respectively, such that the sleeve is arranged in the proximal axial slot and stopped by the bent circumferential bridge section, and the wire runs over an outer surface of the bent circumferential bridge section, along the distal axial slot, and towards the active bending section of the tube.

The wire sleeve may bend radially outwards into the proximal axial slot and abut against a proximal side of the bent circumferential bridge section. At the proximal axial slot, the curvature of the wire sleeve may change, such as to assume a step like shape. The control wire extending from an internal space of the wire sleeve may extend over the outer surface of the bent circumferential bridge section and may curve towards the center line of the body past the distal side of the bent circumferential bridge section.

In some examples, the bent circumferential bridge section of the stop section has a width in the circumferential direction, which is larger than a width of the proximal axial slot and/or the distal axial slot, wherein the stop section in particular forms a substantially cross shaped outline, with the proximal axial slot and the distal axial slot forming one axis of the cross shaped outline, and the bent circumferential bridge section forming another axis of the cross shaped outline.

The proximal cutout section and the distal cutout section may each be substantially T-shaped, with the proximal axial slot and the distal axial slot extending along the "legs" of the proximal cutout section and the distal cutout section, respectively, and the top bars of the proximal cutout section and the distal cutout section facing each other and defining the bent circumferential bridge portion. The width of the proximal axial slot and the distal axial slot may be smaller than a width of the bent circumferential bridge section along the circumferential direction.

The width of the bent circumferential bridge section along the circumferential direction of the body may define a depth towards which the bent circumferential bridge section protrudes into the interior of the body for guiding the control wire extending over the outer surface of the bent circumferential bridge section. The width of the distal axial slot and the proximal axial slot along the circumferential direction may be the same or different. In some examples, the width of the distal axial slot is smaller than the width of the proximal axial slot. The width of the proximal axial slot may be configured for preventing the wire sleeve from skipping over the bent circumferential bridge section towards the distal side of the bent circumferential bridge section. In some examples, the width of the proximal axial slot may be configured for receiving the wire sleeve. In some examples, the width of the proximal axial slot is larger than a radial extension of the wire sleeve.

A length of the proximal axial slot along the axial direction may be configured, such that the wire sleeve bends by at least 10° or 20° with respect to the axial direction of the body, e.g. in a portion of the stop section under the proximal axial slot, when the control wire runs over the bent circumferential bridge section and the wire sleeve abuts against the bent circumferential bridge section. In some examples, the length of the proximal axial slot is configured, such that the wire sleeve bends by at most 45° with respect to the axial direction of the body, when the control wire runs over the bent circumferential bridge section and the wire sleeve abuts against the bent circumferential bridge section. In some examples, the length of the proximal axial slot is smaller than the length of the distal axial slot. In some examples, the length of the distal axial slot along the axial direction may be configured, such that the control wire bends by at most 10° or 20° with respect to the axial direction of the body, when the control wire runs over the bent circumferential bridge section and into the inner channel defined by the body.

The stop section can be integrally formed with a passive bending section, and may feature a higher effective bending rigidity than the bending rigidity of the passive bending section and/or the active bending section, such as to prevent the wire sleeve from skipping over the stop section while the passive bending section and/or the active bending section are strained.

In some examples, the circumferential bridge sections further comprise cuts, which span portions of the circumferential bridge sections between adjacent uncut axial bridges, wherein the cuts feature a width in the axial direction, which is smaller than a width of the cutout sections in the axial direction.

The width of the cuts may be at least two times or at least three times smaller than the width of the cutout sections in the axial direction, e.g. when referring to an average width or maximum width of the cuts and the cutout sections. The width of the cuts may be similar to the wall thickness, such as smaller than twice the wall thickness of the body, such as to define a comparatively small transverse bending mode, e.g. when compared to the main bending mode of the active bending section defined through the arrangement of the cutout sections.

The cuts may be non-continuous at a center of the circumferential bridge section between neighboring cutout sections, e.g. with a section of the circumferential bridge section not cut being shifted about the center axis by a rotation of about 90°.

In some examples, the cutout sections are irregularly spaced along the axial direction in the active bending section.

The spacing of the cutout sections may vary the effective bending stiffness of the active bending section. For example, a bending stiffness of the active bending section may be lower in a distal portion of the active bending section than in a proximal portion of the active bending section. The effective bending stiffness can be adjusted for different use cases by varying the cut pattern of the cutout sections, e.g. by increasing/reducing the frequency of the cutout sections in the body 24 and/or by increasing/reducing the width of the cutout sections along the axial direction. For example, the distal part of the bending section can be softer and the proximal part more rigid.

The spring element nature of the hinges implemented by the uncut axial bridges may apply a return force which tends to push the active bending section back to a natural o° (straight) configuration.

In some examples, the body comprises at least three bent circumferential bridge portions at different positions distributed along the axial direction in the active bending section of the tube for defining at least three corresponding control wire slots at the different positions distributed along the axial direction to guide a control wire.

The insertion tube may, for example and without limitation, be an insertion tube or part thereof, and may be configured for use in a bronchoscope, a sinuscope, a nasopharyngoscope, a laryngoscope, a laparoscope, a gastroscope, a duodenoscope, a colonoscope, an echoendo-scope, a hysteroscope, a cystoscope, a uroscope, a urethroscope, a cardioscope, and an arthroscope. A length, a diameter and/or a rigidity/bendability of the tube may be chosen accordingly.

The body may for example form an insertion tube for use in an endoscope or a part of an insertion tube for use in an endoscope. In one example, the body forms a hypotube, for use in an endoscope. The hypotube may for example be configured to be surrounded or enclosed by a tubular sleeve or cover to form an insertion tube. The hypotube may for example form a frame or skeleton of the insertion tube that is configured to provide dimensional stability to the insertion tube.

The present disclosure further provides an endoscope including a tube according to any one of the examples described herein. The body may form the insertion tube of the endoscope or a part thereof, in particular a hypotube. The endoscope may further include an interface/control portion that is configured to be coupled to the tube.

According to a further aspect, a method of manufacturing a tube for an endoscope is provided. The method comprises providing a hollow cylindrical body for an active bending section of the tube, and cutting a plurality of cutout sections into the cylindrical body, the cutout sections being distributed along the axial direction of the body, such that the body is partially discontinuous at the cutout sections. Uncut axial bridges connect proximal and distal sections of the body adjacent to the cutout sections, and circumferential bridge sections are formed between the cutout sections, the circumferential bridge sections extending continuously around a perimeter of the continuous cylindrical body. The method further comprises bending a subset of the circumferential bridge sections radially inward to form bent circumferential bridge sections between neighboring cutout sections, wherein a lateral extension of the body perpendicular to the axial direction is reduced at the bent circumferential bridge sections.

The hollow cylindrical body may comprise an oval, in particular circular, cross-section, with perimeter walls with a thickness which is smaller than 10% of the diameter of the hollow cylindrical body. For example, the thickness may be 1 mm or smaller, such as 0.5 mm or smaller, or 0.15 mm or smaller.

In some examples, the cutting is performed with a laser, e.g. with the laser tracing an outline of the cutout-sections.

In some examples, the method further comprises guiding a control wire of the tube through wire guidance slots defined between an outer surface of the bent circumferential bridge sections and inner surfaces of neighboring circumferential bridge sections, to guide the control wire along the axial direction of the body for actively angulating the tube.

In some examples, the method further comprises guiding a control wire through a stop section arranged at a proximal portion of the active bending section such that a wire sleeve of the control wire abuts against a bent circumferential bridge section in the stop section to prevent the wire sleeve from moving towards the distal end of the active bending section.

The method may further comprise cutting passive cutout sections into a proximal section of the body, proximal with respect to the active bending section and the stop section, for providing the tube with a passive bending section.

### LIST OF FIGURES

In the following, a detailed description of the present disclosure and examples thereof is given with reference to the figures. The figures show schematic illustrations of
Fig. 1: an endoscope according to an example of the present disclosure;
Fig. 2A: a portion of an active bending section of an insertion tube for an endoscope according to an example of the present disclosure in a first side view;
Fig. 2B: the portion of the active bending section of Fig. 2A in a second side view;
Fig. 2C: a top part of the body in a sectional view along the axial direction of the insertion tube close to a bent circumferential bridge section according to an example;
Fig. 3A: a portion of an insertion tube for an endoscope according to an example of the present disclosure in a straight configuration;
Fig. 3B: the portion of the insertion tube of Fig. 3A in a close-up view focused on the active bending section in a bent configuration;
Fig. 4A: a top view of a stop section of an insertion tube according to an example of the present disclosure;
Fig. 4B: a side view of a stop section of an insertion tube according to an example of the present disclosure;
Fig. 5A: a cross section of an active bending section of an insertion tube according to an example of the present disclosure;
Fig. 5B: a cross section of an active bending section of an insertion tube according to a comparative example; and
Fig. 6: a flowchart of a method of manufacturing an insertion tube according to an example of the present disclosure.

### DESCRIPTION OF EXAMPLES

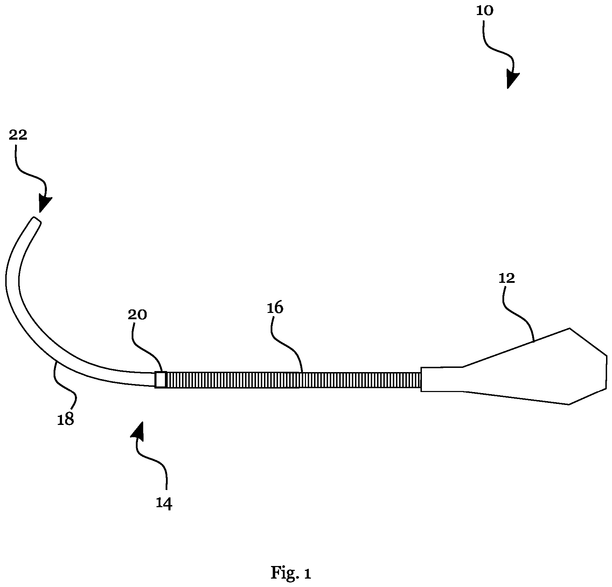

Fig. 1 shows a schematic illustration (not to scale) of an endoscope 10 according to an example of the present disclosure. The endoscope 10 includes an interface/control portion 12 and an insertion tube 14 comprising a passive bending section 16, and an active bending section 18, arranged distally with respect to the passive bending section, with a stop section 20 arranged between the active bending section 18 and the passive bending section 16. The endoscope 10 extends from the interface/control portion 12 to a distal end 22 arranged at a distal side of the active bending section.

The insertion tube 14 may be formed of different tube sections of a generally rigid material, which may be structured to control an effective bending stiffness of the insertion tube 14. A pipe made of stainless steel is particularly preferred. However, a pipe made of hard plastic can also be used. In principle, however, any material suitable for medical purposes can be used.

The insertion tube 14 may be substantially tubular shaped. The insertion tube 14 extends along a center line of the tube 14 from a proximal end adjacent to the interface/control portion 12 (e.g. connected or attached thereto via a connector) to a distal end 22, wherein the distal end 22 is facing away from the interface/control portion 12. A direction X along the center line from the proximal end to the distal end 22 may be referred to as a longitudinal, axial or X direction in the following. A direction φ along a circumference of the tube (e.g. parallel to a surface of the tube and perpendicular to the longitudinal direction X) may be referred to as a circumferential, azimuthal or φ direction in the following.

One or more ports(not shown) may be provided at the interface/control portion 12 and may for example include one or more fluid ports that are in fluid communication with an interior of the insertion tube 14, for example with a channel (not shown) formed by or arranged in the insertion tube 14. Additionally or alternatively, the one or more ports may include one or more light guide ports, wherein each of the light guide ports may for example be configured to receive a light guide (not shown) that is to be arranged within the insertion tube 14 and/or to provide coupling to a light guide (not shown) that is arranged within the insertion tube 14, for example in a channel formed by or arranged in the insertion tube 14. The interface/control portion 12 may further include one or more electrical contacts (not shown), e.g. to provide an electrical connection to the interior of the insertion tube 14, to a distal end 22 of the insertion tube 14 and/or to a distal tip (not shown) of the endoscope 10, which may e.g. be arranged at or connected to the distal end 22 of the insertion tube 14. The interface/control portion 12 may also include means for actuating a distal active bending section 18 of the insertion tube 14, for example one or more control knobs (not shown), which may e.g. be configured to apply tension to one or more control wires (not shown) coupled to the active bending section 18, e.g. via one or more attachment point at the distal end 22.

The interface/control portion 102 may be embodied as a single unit as shown in Fig. 1 or as two or more separate units, for example a control portion and an interface portion, wherein the control portion may e.g. be connected to the proximal end 22 of the insertion tube 14 via a connector and the interface portion may e.g. be connected to the control portion via a flexible tube or an umbilical cord.

The proximal passive bending section 16 may be configured to be bent or deformed when applying an external force. The passive bending sections may for example be made of (e.g. include) a flexible material. Additionally or alternatively, the passive bending sections may be made of a rigid or semi-rigid material such as metal (e.g. stainless steel) and may for example include a plurality of bendable segments, in each of which one or more openings may be provided in a wall of the insertion tube 14 to increase a flexibility of the insertion tube 14 in the respective bendable segment. In some examples, a proximal section of the insertion tube may also include a rigid section (not shown), either in addition to or instead of the one or more passive bending section, e.g. at the proximal end adjacent to a connector to the interface/control portion. A length and a diameter of the insertion tube 14 may be chosen to be suitable for the type of endoscope that the insertion tube 14 is to be used with. The insertion tube 14 may for example have a length between 20 cm and 200 cm, in some examples between 50 cm and 150 cm. An outer diameter of the tube 14 may for example be between 1 mm and 20 mm, in some examples between 2 mm and 10 mm.

In the example of Fig. 1, an active bending section 18 is provided which can be actively angulated based on a controlling force applied through the interface/control portion 12, in particular via control wires passing from the interface/control portion through the passive bending section 16 to the active bending section 18. The default configuration of the active bending section 18 may for example be a straight configuration, in which the insertion tube 14 is not bent and the center line is a straight line. The default configuration may for example be the configuration that the insertion tube 14 ideally (e.g. without any external perturbation) assumes when no additional tension is applied to the control wires, e.g. such that the control wires create a uniform (non-directional) compressive tension in the longitudinal direction. In some examples, the active bending section 18 may be actuated or controlled actively, e.g. using corresponding means for actuating the active bending section 18 such as control wires. When the control wires are tensioned, the active bending section 18 may assume a bent configuration, with the center line following a curved line between a proximal and a distal end of the active bending section 18, as shown in Fig. 1. The control wires may be configured as Bowden cables, and a cable sleeve of the control wires may extend from the interface/control portion 12 of the endoscope 10 to the stop section 20 for limiting a deformation of the passive bending section 16 in response to a tensioning of the control wires.

In the prior art, the active bending section 18 is generally implemented using a plurality of articulated joints, to enable a controlled angulation of the active bending section.

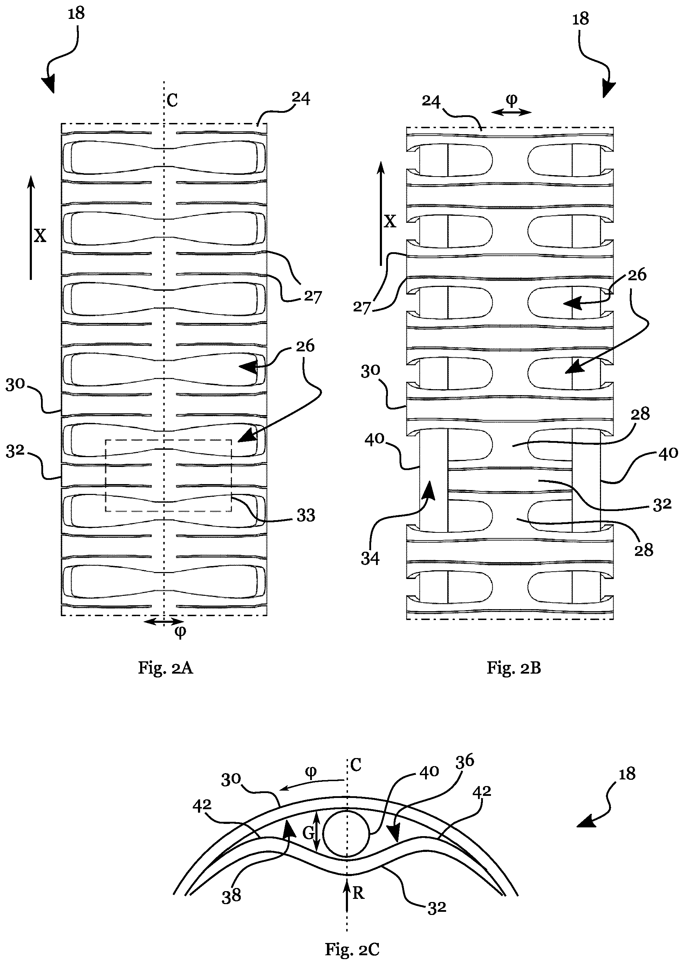

Fig. 2A, 2B illustrate side views of an active bending section 18 according to examples of the present disclosure, with the views rotated by 90° with respect to each other. The active bending section 18 is formed from a continuous cylindrical body 24 extending along an axial direction X. The body 24 defines an inner channel radially delimited by the cylindrical structure. The body 24 may for example be made of (e.g. include, i.e. including, but not limited to) a semi-rigid or rigid material, in particular a metal such as stainless steel, titanium or a nickel-titanium alloy such as Nitinol. In some examples, the body 24 may consist of (i.e. including nothing else but) a semi-rigid or rigid material, in particular a metal such as stainless steel, titanium or a nickel-titanium alloy such as Nitinol.

The body 24 features a plurality of cutout sections 26 distributed along the axial direction X. The cutout sections 26 are designed to create partial discontinuities in the body 24. Uncut axial bridges 28 connect the proximal and distal sections of the body 24 adjacent to the cutout sections 26. The uncut axial bridges 28 maintain the structural integrity of the body 24 while permitting controlled bending with the uncut axial bridges 28 acting as hinges. Circumferential bridge sections 30 are formed between the cutout sections 26, with the circumferential bridge sections 30 arranged at the same position along the axial extension of the body 24 together forming substantially annular sections of the body 24, with neighboring circumferential bridge sections 30 being able to approach or move away from each other by pivoting with respect to an uncut axial bridge 28 connecting the neighboring circumferential bridge sections 30, which may change an axial extension of a cutout section 26 arranged between the neighboring circumferential bridge sections 28.

As illustrated in Figs. 2A-B, an outline of the cutout sections 26 may be elongated along the circumferential direction φ of the body 24, wherein an extension of the cutout sections 26 along the circumferential direction φ may be larger than an extension of the cutout sections 26 along the axial direction X, in particular by a factor of more than two, such as greater than three or greater than four, when the body 24 is in a default configuration (e.g. free of external strain).

In the example, pairs of cutout sections 26 are arranged at the same position along the axial direction X. The cutout sections 26 of each pair are arranged on opposite sides of the cylindrical body 24 in a radial direction R (extending radially from a centerline of the body 24, as illustrated in Fig. 2C). The pairs of cutout sections 26 are separated by uncut axial bridges 28 on opposite sides in the circumferential direction φ. This arrangement may ensure symmetrical bending with the uncut axial bridges 28 acting as hinges and may maintain the tube's structural integrity. At an axial position along the body 24, the cutout sections 26 may together cover more than 70% or more than 80% of the perimeter of the body 24.

As illustrated in the example, the outline of the cutout sections 26 may further feature a dumbbell- or bow-tie-shape, with a center C of the cutout section 26 along the circumferential direction φ featuring a smaller width along the axial direction X than neighboring side portions of the respective cutout section, with the neighboring side portions being shifted with respect to the center C of the respective cutout sections 26 along the circumferential direction φ of the body 24, at least when the body 24 is in a default configuration. Accordingly, adjacent circumferential bridge sections 30 may feature a middle portion with a width along the axial direction X which is larger than a width of the circumferential bridge sections 30 in a circumferential end portion closer to an adjacent uncut axial bridge 28 and/or a circumferential end of a neighboring cutout section 26.

The body 24 further features a plurality of cuts 27, which have a width in the axial direction X smaller than the width of the cutout sections 26 and are arranged in a rotationally shifted position with respect to the cutout sections 26 on the circumferential bridge sections 30. Specifically, the cuts 27 are discontinuous at the center C of the circumferential bridge sections 30, but span the uncut axial bridges 28 along the circumferential direction φ.

The cuts 27 may define a passive transverse bending mode of the active bending section 18, enabling the active bending section to bend in a direction perpendicular to the main bending mode, which can be defined by the cutout sections 26, the uncut axial bridges 28 and the control wire 40. The passive transverse bending mode may reduce a risk of injury to a patient and may facilitate control while the insertion tube is advanced into a biological lumen of a patient. Since the extent of the cuts 27 in the axial direction X is substantially smaller than the corresponding extension of the cutout sections 26, e.g. by a factor of 2 or more, 3 or more, or 4 or more, the active bending section may be primarily angulated along the main bending mode, i.e. with the uncut axial bridges 28 acting as hinges.

A subset of the circumferential bridge sections 30 is bent radially inward, e.g. by pressing with a tool onto a bending section 33, forming bent circumferential bridge sections 32 between neighboring cutout sections 26. The bending can reduce the lateral extension of the body 24 perpendicular to the axial direction X at the bent circumferential bridge sections 32. The lateral extension may be defined by the distance between opposite sides of the body 24 along a line through a center of the bent circumferential bridge sections 32 and through the center line of the body 24. The bent circumferential bridge sections 32 can be formed by applying localized mechanical strain on the (circumferential) center C of the bent circumferential bridge sections 32 to displace the center C of the bent circumferential bridge sections 32 towards the center line of the body 24, i.e. into a substantially cylindrical cavity enclosed by an inner wall of the body 24.

Fig. 2C illustrates a view of a top part of the body 24 along the axial direction X close to the bent circumferential bridge sections 32. At the bent circumferential bridge section 32, the circumferential bridge sections 30 can create a wire guidance slot 34 between an outer surface 36 of the bent circumferential bridge sections 32 and the inner surfaces 38 of neighboring circumferential bridge sections 30. These wire guidance slots 34 can guide a control wire 40, which can be used to actively angulate the insertion tube 14, and is shown in Figs. 2B and 2C.

Specifically, the control wire 40 can run between the outer surface 36 of the bent circumferential bridge sections 32 and the inner surfaces 38 of neighboring circumferential bridge sections 30. As shown in the example, based on the dumbbell shaped outline of the neighboring cutout sections 26, the bent circumferential bridge section 32 may be bent at two intermediate bending portions 42 arranged on opposite sides of the center C of the bent circumferential bridge section 32, and the cross section of the bent circumferential bridge section 32 maybe V- or W-shaped when viewed along the axial direction X (into the plane of projection of Fig. 2C).

A radial gap G between the outer surface 36 of the bent circumferential bridge sections 32 and the inner surfaces 38 of neighboring circumferential bridge sections 30 may be largest close to a center C of the bent circumferential bridge section 32, and the control wire 40 may be biased towards the center of the wire guidance slot 34. As the control wire 40 is supported by the outer surface 36 of the bent circumferential bridge section 32, which conforms to the cylindrical surface of the body 24 depressed towards the center line, the control wire 40 can be supported over an extended axial section of the body 24 along the width of the bent circumferential bridge section 32, which can allow reducing the material thickness of the body 24, when compared to conventional active bending section geometries, in which control wires 40 are generally guided with through-holes of inward bent flaps such that the control wire 40 would be supported along the thickness direction of the original cylindrical body 24.

As illustrated in Fig. 2B, two control wires 40 may be provided on opposite side of the body 24 along the radial direction R to actively bend the active bending section 18 in two opposite directions. Specifically, by pulling on one of the control wires 40 arranged on one side of the body 24 and by optionally relaxing the control wire 40 arranged on the other side of the body 24, the active bending section may be urged to bend towards the pulled control wire 40, with the uncut axial bridges 28 being deformed to mediate a hinge-like bending of the active bending section 18.

The skilled person will appreciate that although two wires 40 are shown in Fig. 2B, the illustrated control wires 40 may be sections of the same wire, e.g. connected at the distal end of the active bending section 18, or only a single control wire 40 may be provided in examples, e.g. on one side of the body 24, displaced with respect to a center line of the body 24.

Outside of the active bending section 18, the control wire 40 may be sheathed in a wire sleeve, such as to localize a bending strain transmitted through the control wire 40 to the active bending section 18.

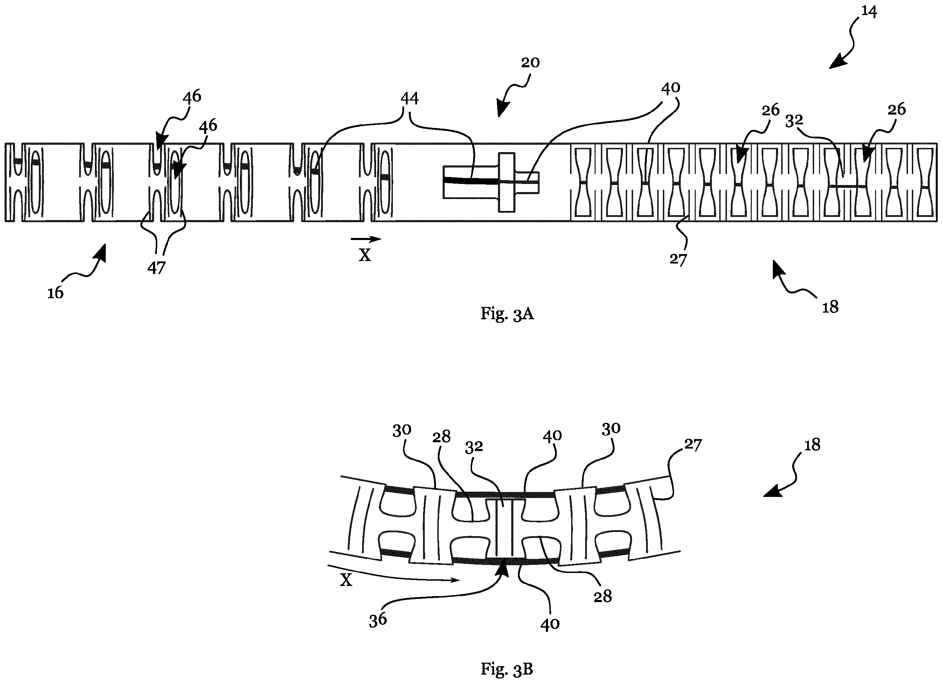

Fig. 3A illustrates a portion of an insertion tube 14 including a passive bending section 16 and an active bending section 18 with a stop section 20 arranged therebetween. In the illustrated example, the insertion tube 14 is formed from a continuous cylindrical body 24 with the passive bending section 16, the active bending section 18, and the stop section 20 being integrally formed through cutting of the continuous cylindrical body 24. However, in other examples, the active bending section 18 may only be integrally formed with the stop section 20, while the passive bending section is joined to the stop section 20, or vice-versa.

A control wire 40 runs along the axial direction X of the insertion tube 14 inside of an inner channel of the body 24 through the passive bending section 16, the active bending section 18, and the stop section 20. The control wire 40 is sheathed with a wire sleeve 44 in the passive bending section 16 up to the stop section 20, and is not sheathed in the active bending section 18. The wire sleeve 44 is stopped via axial abutment in the stop section 20, such as to prevent further advance of the wire sleeve 44 towards the active bending section 18.

Fig. 3B illustrates a side view of a portion of the active bending section 18. Two control wires 40 are arranged on opposite sides of the body 24 from which the active bending section 18 is formed, with the upper control wire 40 subjected to a larger amount of pulling tension than the bottom control wire 40. As a result, the active bending section is bent, with the circumferential bridge sections 30, 32 pivoting with respect to each other, while the uncut bridge sections 28 act as hinges, the uncut bridge sections 28 being shifted with respect to a control wire 40 path by about π/2 about the center line of the body 24.

In the bent configuration illustrated in Fig. 3B, the upper control wire 40 abuts against inner surfaces 38 of the adjacent circumferential bridge sections 30, to transmit a pulling force, e.g. applied to the control wire 40 on proximal ends of an endoscope 10, to the body 24 of the active bending section 18. At the same time, the other (bottom) control wire 40 abuts against the outer surface(s) 36 of the bent circumferential bridge section(s) 32 to prevent the other control wire 40 from further extending into the inner channel of the body 24 while it is in a less tensioned state.

The pulling tension applied along the control wires 40 deforms the body 24 to deform according to a bending in an angulation plane, which coincides with the plane of projection of Fig. 3B. The angulation plane can run through the two control wires 40, and may be angled by about π/2 with respect to a plane running through the center line of the body 24 and through the uncut axial bridges 28 on opposite sides of the body 24 about the axial direction X.

As illustrated in Fig. 3B, the axial edges of neighboring circumferential bridge sections 30 approach each other on the side of the tensioned control wire 40 and are distanced from each other on the opposite side, effectively shortening/lengthening the cutout sections 26 on the respective sides in the axial direction X. The width of the circumferential bridges sections 30, 32 in the axial direction X may limit a maximum relative tilt angle between neighboring circumferential bridge sections 30, 32. When the width of the circumferential bridges sections 30, 32 in the axial direction X is increased closer to the center C of the cutout sections 26, wire guidance of the control wire 40 may be increased without limiting the bending stiffness of the active bending section 18.

In the passive bending section 16, the body 24 is equally patterned with passive cutout sections 46 and additional cuts 47, to control a bending stiffness of the passive bending section 16. As illustrated in Fig. 3A, the outline of the passive cutout sections 46 may be different from the cutout sections 26 in the active bending section 18, e.g. to provide a different bending stiffness in the two sections 16, 18. As shown in Fig. 3B, the passive cutout sections 46 may feature an oval outline, e.g. an elliptic, superelliptic or rectangular outline, whereas the cutout sections 26 in the active bending section 18 may feature a dumbbell- or bow-tie-shape. Additional tuning of the bending stiffness in the passive bending section can be provided by the cuts 47, which may be similar to the cuts 27 of the active bending section 18.

In the passive bending section 16, the passive cutout sections 46 are arranged in pairs along the axial direction X, with every other passive cutout section 46 being rotated by about π/2 the axial direction X from the next, such as to enable substantially equal bending stiffness of the body 24 in two perpendicular passive bending directions. Although this configuration is only shown for the passive bending section 16, the skilled person will appreciate that a similar configuration may also be provided in the active bending section 18, e.g. with four instead of two control wires 40. Thus, the active bending section 18 may also feature two angulation planes for bending the active bending section 18 according to two perpendicular active bending modes, based on the modulation of a pulling strain on associated (pairs of) control wires 40. Corresponding pairs of uncut axial bridges 28, defined by respective pairs of cutout sections at a specific axial position and aligned perpendicular to one of the two active bending modes, may be staggered along the axial direction X, e.g. to define multiple hinges for the respective active bending modes along the axial direction X.

Active bending of the passive bending section 16 in response to a tensioning of the control wires 40 may be prevented by the wire sleeve 44 through which the control wires 40 are guided along the axial extension of the passive bending section 16. The wire sleeve 44 may be fixedly connected to a proximal end of the passive bending section 16 and abuts against an abutment portion of the stop section 20, as shown in Fig. 3B at a distal end of the passive bending section 16 and close to a proximal end of the active bending section 18.

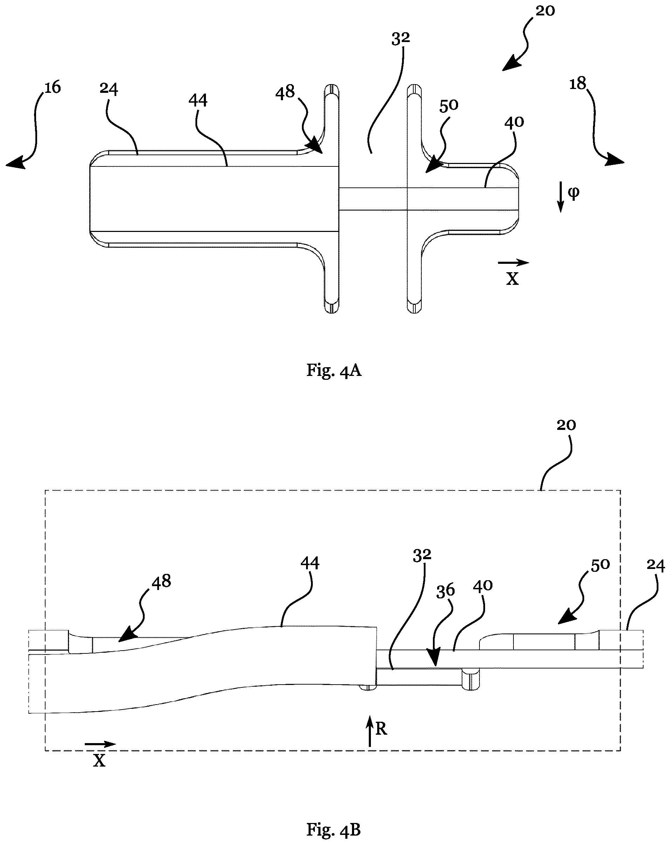

Figs. 4A and 4B illustrate close-up views of a stop section 20 with a wire sleeve 44 of a control wire 40 abutting against a bent circumferential bridge section 32 defined by adjacent cutout sections 26 in the body 24 and bent inwards towards a center line of the body 24 according to a top view and a sectional side view of the stop section 20, respectively.

The cutout sections 26 comprise a proximal cutout section 48 arranged proximally with respect to the bent circumferential bridge section 32 and a distal cutout section 50 arranged distally with respect to the circumferential bridge section 32. The control wire 40 runs over the outer surface 36 of the bent circumferential bridge section 32 and back into the inner channel of the body 24 delimited by the inner wall of the adjacent section of the body 24.

However, the wire sleeve 44 abuts against a proximal axial edge of the bent circumferential bridge section 32 of the stop section 20 to prevent the wire sleeve 44 of the control wire 40 running through the stop section 20 from moving towards the active bending section 18.

Both the proximal cutout section 48 and the distal cutout section 50 feature a T-shaped outline, wherein a leg of the cutout sections 48, 50 (corresponding to the vertical bar of the letter "T") extends along the axial direction X of the body 24 and a top bar section of the cutout sections 48, 50 (corresponding to the horizontal bar of the letter "T") extending along the circumferential direction φ. The legs of the proximal and distal cutout sections 48, 50 may form proximal and distal axial slots, respectively, extending up to the bent circumferential bridge section. The top bar sections of the cutout sections 48, 50 face each other and may axially delimit the bent circumferential bridge section 32 of the stop section 20. As illustrated in Figs. 4A, 4B an extension of the distal cutout section 50 in the axial direction X can be smaller than the axial extension of the proximal cutout section 48, in particular with the leg of the distal cutout section 50 being shorter than the leg of the proximal cutout section 48 in the axial direction X.

Moreover, a width of the leg of the proximal cutout section 48 in the circumferential direction φ may be greater than a width of the leg of the distal cutout section 50 in the circumferential direction φ, and may be configured to prevent the wire sleeve 44 from advancing through the distal cutout section 50 into the inner channel of the body 24, if the wire sleeve 44 inadvertently skips over the bent circumferential bridge section 32 of the stop section 20.

The width of the leg of the proximal cutout section 48 may be configured to be greater than a diameter of the wire sleeve 44, e.g. such as to allow the wire sleeve 44 in the stop section 20 to curve away from the center line of the body 24 along the radial direction R towards the proximal cutout section 48. As illustrated in Fig. 4B, the wire sleeve may bend by more than 10°, with respect to the axial direction X, such as to enable the wire sleeve 44 to move into the proximal cutout section 48 and to minimize friction of the control wire 40 running over the bent circumferential bridge section 32 of the stop section, e.g. when the control wire 40 is pulled with respect to the wire sleeve 44 to adjust a bending state of the active bending section 18. By limiting the length of the leg of the proximal cutout section 48 the emerging angle of the wire sleeve 44 emerging from the inner channel of the body 24 proximal to the stop section 20 may be controlled to be above 10°. An emerging angle above 10° or above 20° may reduce the risk of the wire sleeve 44 bowing outward and skipping over the bent circumferential bridge section 32. The emerging angle of the wire sleeve 44 emerging from the inner channel of the body 24 proximal to the stop section 20 should be below 45° to protect the control wire 40 from large external loads.

At the same time, the length of the distal cutout section 50 may be smaller than the length of the proximal cutout section 48, such as bias the control wire 40 emerging from the wire sleeve 44 towards the center line of the body 24, e.g. to hold down the wire sleeve 44 in the stop section 20, when the control wire 40 is tensioned.

The width of the top bar section of the cutout sections 48, 50 of the stop section 20 in the circumferential direction φ may be similar or smaller than a corresponding width of cutout sections 26 in the active bending section 18, e.g. to limit a radial gap G created by bending the bent circumferential bridge section 32 towards the center line of the body 24.

Preferably, an angle of the control wire 40 with respect to the center line of the body 24 in a distal portion of the stop section 20, i.e. distal with respect to the bent circumferential bridge section 32, is smaller than 20° for reducing wire friction in the stop section 20. The angle may be controlled through providing a sufficient length of the leg of the distal cutout section 50 and/or the radial gap G created by bending the bent circumferential bridge section 32 in the stop section 20.

The stop section 20 with a bent circumferential bridge section 32 for limiting an advance of the wire sleeve 44 towards the active bending section 18 may provide a robust assembly, which may increase an effective diameter of an inner channel of the body 24, e.g. for accommodating components of an endoscope 10.

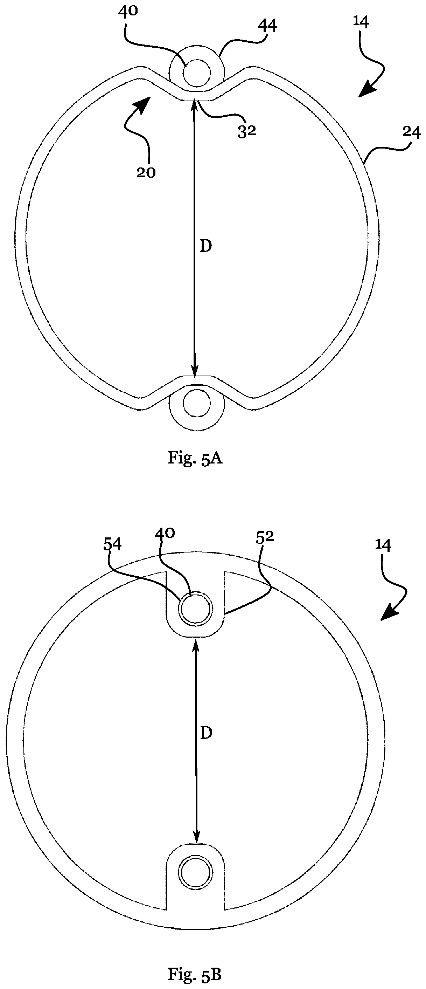

Fig. 5A illustrates a cross-sectional view of a stop section 20 of an endoscope 10 along the axial direction X according to an example of the present disclosure and Fig. 5B illustrates a comparative example of a stop section which stops a wire sleeve 44 of a control wire 40 using inward bent flaps 52 of a hypotube body 24 with a through hole 54 for passing the control wire 40 and stopping the wire sleeve 44. As can be seen from a comparison of Figs. 5A, 5B, a maximum diameter D of an inscribed circle in the inner channel of the body 24 is increased for the example of the present disclosure with respect to the comparative example with flaps 52 for guiding the control wire 40 and for providing the functionality of the stop section 20 of limiting the advance of the wire sleeve 44. Moreover, since the strain of the wire sleeve 44 abutting against the bent circumferential bridge section 32 is applied along a direction, which is perpendicular to the thickness direction of the material of the body 24 (the radial direction R), the thickness of the body 24 may be reduced without compromising the structural integrity of the stop section 20. In some examples, the thickness of the cylindrical wall of the body 24 in the radial direction R is smaller than 0.15 mm.

Thus, the active bending section 18 and the stop section as described above can allow omitting flaps and puzzled hinges, and may provide more space in diameter, more flexible arrangement of inner materials, and larger space for a working channel for an endoscope. At the same time, a lower material thickness limit for the active bending section 18 and/or the stop section 20 may be relaxed.

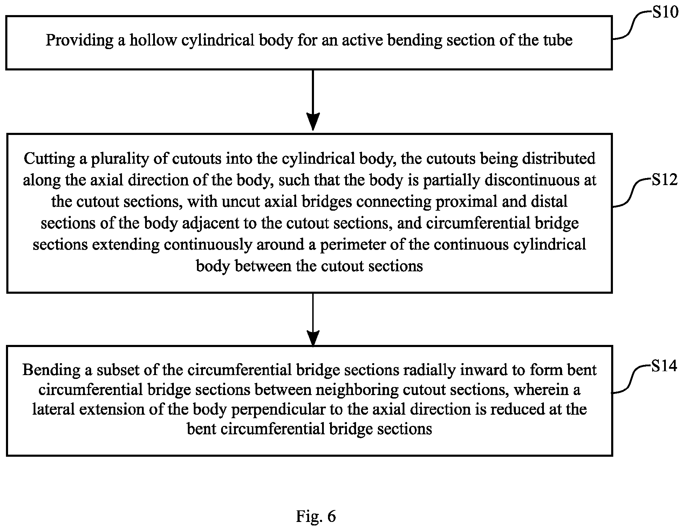

Fig. 6 illustrates a flowchart of a method for manufacturing a tube 14 for an endoscope 10 according to an example of the disclosure. The method comprises providing (S10) a hollow cylindrical body 24 for an active bending section 18 of the tube 14. The method further comprises cutting (S12) a plurality of cutout sections 26 into the cylindrical body 24, the cutout sections 26 being distributed along the axial direction X of the body 24, such that the body 24 is partially discontinuous at the cutout sections 26, with uncut axial bridges 28 connecting proximal and distal sections of the body 24 adjacent to the cutout sections 26, and circumferential bridge sections 30 extending continuously around a perimeter of the continuous cylindrical body 24 between the cutout sections 26. The method further comprises bending a subset of the circumferential bridge sections 30 radially inward to form bent circumferential bridge sections 32 between neighboring cutout sections 26, wherein a lateral extension of the body 24 perpendicular to the axial direction X is reduced at the bent circumferential bridge sections 32.

Cuts can be provided in the hollow cylindrical body 24 by a laser cutting machine, and may be used to form the cutout sections 26 according to a pre-programmed cutting pattern. After providing the cuts, certain parts of the hollow cylindrical body 24 can be bent by mechanically pressing on the outer surface of the cylindrical body 24. For example, a tool may be used to press on a bending section 33 to bend a subset of the circumferential bridge sections 30 inward, e.g. to form wire guidance slots 34 and/or a stop section 20 in a portion of the body 20. Further cuts 27, 47 may be provided along the length of the body 24 in the axial direction, such as to tune a bending stiffness of the body 24 with respect to different (passive) bending modes of the tube 14 formed using the body 24. In some examples, a single tube is used to form a passive bending section 16, an active bending section 18, and a stop section 20 from a common body 24 by varying the cutting pattern, such as to substantially manufacture a hypotube of a tube 14 from a single piece of a base body 24. In other examples, the different sections 16, 18, and 20 of the hypotube are formed individually from different tube parts, and are connected to each other, e.g. after cutting and/bending the respective tube parts. In some examples, at least the active bending section 18 and the stop section 20 are formed from the same hollow tube.

Thereafter, the base body 24 for the tube 14 may be provided with a control wire 40 and surrounded (sheathed) with a cover element (sheath element, not shown) for manufacturing an insertion tube 14 for an endoscope 10. The insertion tube 14 may be joined to an interface/control portion 12 and components of an endoscope 10 may be inserted into the inner channel of the body 24 for assembling the endoscope 10.

The examples of the present disclosure disclosed herein only constitute specific examples for illustration purposes. The present invention can be implemented in various ways and with many modifications without altering the underlying basic properties. Therefore, the present invention is only defined by the claims as stated below.

## Claims

1. A tube (14) for an endoscope (10), the tube (14) comprising a continuous cylindrical body (24) extending along an axial direction (X) and defining an inner channel radially delimited by the body (24),
the body (24) comprising a plurality of cutout sections (26, 48, 50) distributed along the axial direction (X) in an active bending section (18) of the tube (14), wherein the body (24) is partially discontinuous at the cutout sections (26, 48, 50), such that uncut axial bridges (28) connect proximal and distal sections of the body (24) adjacent to the cutout sections (26, 48, 50), and wherein circumferential bridge sections (30, 32) of the body (24) are formed between the cutout sections (26, 48, 50) along the axial direction (X);
wherein a subset of the circumferential bridge sections (30, 32) is bent radially inward to form bent circumferential bridge sections (32) between neighboring cutout sections (26), wherein a lateral extension of the body (24) perpendicular to the axial direction (X) is reduced at the bent circumferential bridge sections (32).

2. The tube (14) of claim 1, wherein the circumferential bridge sections (30, 32) define wire guidance slots (34) between an outer surface (36) of the bent circumferential bridge sections (32) and inner surfaces (38) of neighboring circumferential bridge sections (30), to guide a control wire (40) for actively angulating the tube (14);
wherein in particular two bent circumferential bridge sections (32) are arranged on opposite sides of the cylindrical body (24) in a radial direction (R), to define wire guidance slots (34) symmetrically disposed on opposite sides of the cylindrical body (24).

3. The tube (14) of claim 1 or 2, wherein pairs of cutout sections (26) are distributed along the axial direction (X), the cutout sections (26) of a pair of cutout sections (26) being arranged on opposite sides of the cylindrical body (24) in a radial direction (R), and wherein the cutout sections (26) of the pair of cutout sections (26) are separated by uncut axial bridges (28) on opposite sides of the cutout sections (26) in the circumferential direction (φ).

4. The tube (14) of any one of the preceding claims, wherein the cutout sections (26) comprise an angulation control portion, wherein the angulation control portion is arranged between circumferential ends of the cutout sections (26) and features a width along the axial direction (X), which is smaller than a width of neighboring portions closer to the circumferential ends of the cutout sections (26).

5. The tube (14) of any one of the preceding claims, wherein the cutout sections (26, 48, 50) at an axial position along the body cover more than 70% or more than 80% of the perimeter of the body (24).

6. The tube (14) of any one of the preceding claims, wherein the tube (14) further comprises a control wire (40) extending axially through the active bending section (18) towards a distal end (22) of the active bending section (18), wherein pulling on a proximal portion of the control wire (40) bends the active bending section (18) of the tube (14) with sections of the body (24) pivoting about the uncut axial bridges (28).

7. The tube (14) of any one of the preceding claims, wherein the tube (14) further comprises a stop section (20) arranged at a proximal portion of the active bending section (18) and configured to prevent a wire sleeve (44) of a control wire (40) running through the stop section (20) from moving towards the distal end (22) of the active bending section (18), wherein the wire sleeve (44) abuts against a bent circumferential bridge section (32) in the stop section (20).

8. The tube (14) of any one of the preceding claims, wherein the stop section (20) comprises a bent circumferential bridge section (32) arranged between a proximal axial slot and a distal axial slot defined by a proximal cutout section (48) and a distal cutout section (50), respectively, such that the sleeve is arranged along the proximal axial slot and stopped by the bent circumferential bridge section (32), and the control wire (40) runs over an outer surface (36) of the bent circumferential bridge section (32), along the distal axial slot, and towards the active bending section (18) of the tube (14);
wherein the bent circumferential bridge section (32) of the stop section (20) in particular has a width in the circumferential direction (φ), which is larger than a width of the proximal axial slot and/or the distal axial slot, wherein the stop section (20) preferably forms a substantially cross shaped outline, with the proximal axial slot and the distal axial slot forming one axis of the cross shaped outline, and the bent circumferential bridge section (32) forming another axis of the cross shaped outline.

9. The tube (14) of any one of the preceding claims, wherein the circumferential bridge sections (30, 32) further comprise cuts (27, 47), which span portions of the circumferential bridge sections (30, 32) between adjacent uncut axial bridges (28), wherein the cuts (27, 47) feature a width in the axial direction (X), which is smaller than a width of the cutout sections (26) in the axial direction (X).

10. The tube (14) of any one of the preceding claims, wherein the cutout sections (26) are irregularly spaced along the axial direction (X) in the active bending section (18).

11. The tube (14) of any one of the preceding claims, wherein the body (24) comprises at least three bent circumferential bridge portions (32) distributed along the axial direction (X) in an active bending section (18) of the tube (14) for defining at least three corresponding angulation wire slots (34) to guide a control wire (40).

12. An endoscope (10) comprising the tube (14) according to any one of the preceding claims, in particular as part of an insertion tube (14).

13. A method of manufacturing a tube (14), wherein the method comprises:
providing a hollow cylindrical body (24) for an active bending section (18) of the tube (14);
cutting a plurality of cutout sections (26, 48, 50) into the cylindrical body (24), the cutouts being distributed along the axial direction (X) of the body (24), such that the body (24) is partially discontinuous at the cutout sections (26, 48, 50), wherein uncut axial bridges (28) connect proximal and distal sections of the body (24) adjacent to the cutout sections (26, 48, 50), and wherein circumferential bridge sections (30) are formed between the cutout sections (26, 48, 50), the circumferential bridge sections (30) extending continuously around a perimeter of the continuous cylindrical body (24);
bending a subset of the circumferential bridge sections (30, 32) radially inward to form bent circumferential bridge sections (32) between neighboring cutout sections (26), wherein a lateral extension of the body (24) perpendicular to the axial direction (X) is reduced at the bent circumferential bridge sections (32).

14. The method of claim 13, wherein the cutting is performed with a laser.

15. The method of claim 13 or 14, wherein the method further comprises guiding a control wire (40) of the tube (14) through wire guidance slots (34) defined between an outer surface (36) of the bent circumferential bridge sections (32) and inner surfaces (38) of neighboring circumferential bridge sections (30), to guide the control wire (40) along the axial direction (X) of the body (24) for actively angulating the tube (14); and/or
wherein the method further comprises guiding a control wire (40) through a stop section (20) arranged at a proximal portion of the active bending section (18) such that a wire sleeve (44) of the control wire (40) abuts against a bent circumferential bridge section (32) in the stop section (20) to prevent the wire sleeve (44) from moving towards the distal end (22) of the active bending section (18).
